# EUROPEAN PATENT APPLICATION

(11) **EP 4 491 605 A1**
(43) Date of publication of application: **15.01.2025**
(21) Application number: 23184919.1
(22) Date of filing: 12.07.2023
(51) Int. Cl.: C07C 29/78, C07C 31/02

(54) **COMPOSITIONS AND METHODS OF ISOLATING POLICOSANOL AND STEROLS**

(71) Applicant: Amyris Bio Products Portugal, Unipessoal, LDA, 4169-005 Porto (PT); Universidade Católica Portuguesa, 4169-005 Porto (PT)
(72) Inventor: RODRÍGUEZ ALCALÁ, Luis Miguel, 4169-005 Porto (PT); COSTA, Paula, 4169-005 Porto (PT); FONTES, Ana Luiza, 4169-005 Porto (PT); PIMENTEL, Lígia, 4169-005 Porto (PT); PINTADO, Paula, 4169-005 Porto (PT); SOARES, Ana M. S., 4169-005 Porto (PT); TEIXEIRA, Francisca, 4169-005 Porto (PT)
(74) Representative: Nederlandsch Octrooibureau

(57) **Abstract**

The present disclosure provides compositions and methods of recovering a policosanol or a sterol from a mixture comprising one or more fatty alcohols, aldehydes, fatty acids, and/or sterols. The mixture may be, for example, a lipid extract obtained from industrial fermentation processes (e.g., filter cake, distillate residue). The policosanol and/or sterol fraction recovered may be enriched in certain fatty alcohols (e.g., 1-octacosanol, 1-triacontanol, and/or 1-dotriacontanol), or enriched in certain sterols (e.g., ergosterol, campesterol, stigmasterol, and/or β-sitosterol). These bioactive molecules may be useful in the treatment or prevention of various metabolic conditions (e.g., obesity, dyslipidemia, cardiovascular conditions, and other chronic metabolic diseases).

## Description

### FIELD OF THE INVENTION

The present disclosure relates to compositions and methods of purifying and recovering a policosanol and/or a sterol from a lipid composition, such as lipid extracts obtained from industrial sugarcane processing and fermentation waste streams.

### BACKGROUND

Policosanol and sterols are bioactive molecules that have applications in treating and preventing obesity, dyslipidemia, cardiovascular conditions, and other chronic metabolic diseases.

Policosanol is a mixture of fatty alcohols, where 1-octacosanol can be the main component. Policosanol has been shown to reduce body weight gain, fat depots, serum triglycerides, total cholesterol, and low-density lipoprotein (LDL) cholesterol, while increasing high-density lipoprotein (HDL) cholesterol, in animal models of diet-induced obesity. Similar results have been reported for healthy women subjects treated with policosanol. Moreover, hepatoprotective, anti-diabetic, antiinflammatory, antioxidant, anti-carcinogenic, and neuroprotective effects have also been associated with policosanol/1-octacosanol administration.

Sterols associated with health benefits include ergosterol, β-sitosterol, campesterol, and stigmasterol. Ergosterol treatment has been reported to decrease body weight, serum triglycerides, total cholesterol, LDL cholesterol, and increase HDL cholesterol in diet-induced obese mice. Furthermore, ergosterol increases vitamin D bioavailability and possesses anti-carcinogenic properties. Campesterol, stigmasterol, and β-sitosterol have been described to exhibit antiinflammatory and/or anti-cancer activities. In addition, β-sitosterol has demonstrated an anti-diabetic effect.

Policosanol is widely distributed in nature, being found, for instance, in rice bran, wheat, beeswax, and sugarcane. Ergosterol is a mycosterol present in fungi, while β-sitosterol, campesterol, and stigmasterol are phytosterols found in plants. However, recovering these bioactive compounds from their natural sources, in an era where natural resources are over-exploited, would lead to sustainability issues. Therefore, there remains a need to obtain policosanol and sterols in an efficient and inexpensive manner from readily available sources.

In the field of synthetic biology, fermentation waste streams provide a rich source of materials that can be valorized in an environmentally and economically advantageous manner. For example, policosanol and sterols can be isolated from both raw material processing (*e.g.* sugarcane feedstock) as well as from fermentation and/or distillation processes.

Known methods for extracting lipids from fermentation waste streams include, for instance, saponification, solvent extraction (with organic solvents such as hexane, ethanol, or others), sometimes assisted, for improved efficiency, by ultrasound or microwave-based technologies, transesterification, molecular distillation, or supercritical carbon dioxide extraction. The purification of lipid extracts is usually carried out by different kinds of chromatography, namely, thin layer chromatography (TLC), column chromatography, and preparative high-performance liquid chromatography (HPLC). However, these techniques require complex instrumentation and are solvent- and time-intensive. Hence, there is a need in the art for a simpler, faster, and more costeffective purification methods.

### SUMMARY OF THE INVENTION

Provided herein are compositions and methods of purifying and recovering a policosanol or a sterol from lipid compositions, including lipid extracts obtained from synthetic biology waste streams, including sugarcane processing (*e.g.,* filter cake) and fermentation waste streams (*e.g.,* distillate residues). The compositions and methods disclosed herein can be used to valorize such waste streams as part of a circular economy model. The present invention has the advantages of being simple, time and cost effective, and sustainable. For example, it does not require the use of expensive equipment. The present invention relies instead on a winterization process using only a single food grade solvent and cooling conditions.

In a first aspect, the present disclosure provides a method of recovering a policosanol or a sterol from a composition, wherein the method includes: (a) dissolving the composition with a solvent, thereby forming a solution; (b) cooling the solution, thereby causing the policosanol or the sterol to precipitate from the solution; and (c) isolating the policosanol or the sterol, wherein the composition is a mixture comprising one or more fatty alcohols, aldehydes, fatty acids, and/or sterols.

In one embodiment, the solvent is ethyl acetate. In another embodiment, the ratio of the composition to the solvent is between 1:10 (w/v) to 1:30 (w/v). In a further embodiment, the ratio of the composition to the solvent is 1:20 (w/v).

In an embodiment, the solution is cooled to a temperature between 0 °C to -80 °C. In another embodiment, the solution is cooled to a temperature between -10 °C and -60 °C. In a further embodiment, the solution is cooled to -30 °C.

In one embodiment, isolating the policosanol or the sterol occurs by way of a filtration step. In one embodiment, the filtration step comprises a vacuum. In another embodiment, isolating the policosanol or the sterol occurs at a temperature of between -30 °C to 20 °C.

In a further aspect, the present disclosure provides a composition obtained by the methods described herein.

In an embodiment, the composition contains a higher percentage of policosanol or sterol compared to a composition prior to undergoing the recovery methods described herein. In another embodiment, the composition contains 1 to 20 times more policosanol or sterol compared to a composition prior to undergoing the recovery methods described herein. In a further embodiment, the composition contains 2 to 15 times more policosanol or sterol compared to a composition prior to undergoing the recovery methods described herein. In yet another embodiment, the composition contains 3 to 11 times more policosanol or sterol compared to a composition prior to undergoing the recovery methods described herein.

In one embodiment, the policosanol contains fatty alcohols. In another embodiment, the policosanol contains 1-octacosanol, 1-triacontanol, and/or 1-dotriacontanol.

In an embodiment, the sterol contains ergosterol, campesterol, stigmasterol, and/or β - sitosterol.

In one embodiment, the policosanol has a melting point of between 25-30 °C. In another embodiment, the policosanol has a melting point of 28 °C. In yet another embodiment, the sterol has a melting point of between 140-150 °C. In yet another embodiment, the sterol has a melting point of 146 °C.

In an embodiment, the policosanol has a crystallization point of between 15-20 °C. In an embodiment, the policosanol has a crystallization point of between 17-18 °C. In a further embodiment, the sterol has a crystallization point of between 140-150 °C. In another embodiment, the sterol has a crystallization point of 140 °C.

In a further aspect, the present disclosure provides a method for treating or preventing a metabolic condition. The method includes administering to a subject an effective amount of any of the composition described herein. In one embodiment, the metabolic condition is obesity, dyslipidemia, diabetes, or a cardiovascular condition.

### BRIEF DESCRIPTION OF THE DRAWINGS

**FIG. 1** depicts a photographic scheme of an exemplary isolation procedure: A - Lipid extract; B - Lipid extract dissolved in EtAc food grade at 1:20 (w/v); C - Lipid solution after cooling at -30 °C overnight; D - Separation and washing of the isolate from the EtAc (fg) solution by filtration under vacuum; E - Final product after drying at 60 °C.
**FIG. 2** depicts an overlay of exemplary GC-MS chromatograms of PFC vs PEF and respective compositions. PFC: Purified Filtercake, PEF: Policosanol Enriched Fraction.
**FIG. 3** depicts an overlay of exemplary GC-MS chromatograms of PFDR vs SEF and respective compositions. PFDR: Purified Farnesene Distillation Residue, SEF: Sterols Enriched Fraction.
**FIG. 4** depicts an exemplary quantitative composition (%) of PEF (A) and an exemplary comparison of policosanol purity (%) in PFC vs PEF (B). PEF: Policosanol Enriched Fraction, PFC: Purified Filtercake.
**FIG. 5** depicts an exemplary quantitative composition (%) of SEF (A) and comparison of sterols purity (%) in PFDR vs SEF (B). SEF: Sterols Enriched Fraction, PFDR: Purified Farnesene Distillation Residue.
**FIG. 6** depicts an overlay of an exemplary FTIR-ATR spectra of PFC (solid line) and PEF (dashed line). PFC: Purified Filtercake, PEF: Policosanol Enriched Fraction.
**FIG. 7** depicts structures of the main fatty alcohols present in an exemplary Policosanol Enriched Fraction (PEF).
**FIG. 8** depicts an overlay of an exemplary FTIR-ATR spectra of PFDR (solid line) and SEF (dashed line). PFDR: Purified Farnesene Distillation Residue, SEF: Sterols Enriched Fraction.
**FIG. 9** depicts structures of the main sterols present in an exemplary Sterols Enriched Fraction (SEF).
**FIG. 10** depicts the DSC analysis scans of PFC, PEF, PFDR and SEF.

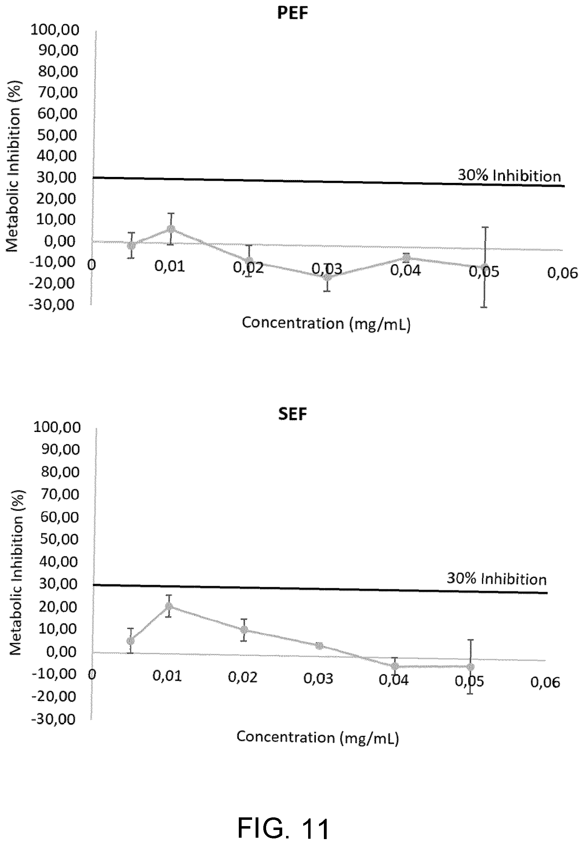

**FIG. 11** depicts the cytotoxicity of an exemplary Policosanol Enriched Fraction (PEF) and an exemplary Sterols Enriched Fraction (SEF) on a HepG2 cell line.

### DETAILED DESCRIPTION

The present disclosure provides compositions and methods of purifying and recovering a policosanol or a sterol from lipid compositions, such as lipid extracts obtained from synthetic biology waste streams, including sugarcane processing waste streams (*e.g.,* filter cake) and fermentation waste streams (*e.g.,* distillate residues). The methods described herein are simple, time and cost effective, and sustainable. By valorizing industrial waste streams, the compositions and methods disclosed herein can contribute to the circular economy model.

### Definitions

As used herein, the singular forms "a," "an," and "the" include plural reference unless the context clearly dictates otherwise.

As used herein, the term "about" when modifying a numerical value or range herein includes normal variation encountered in the field, and includes plus or minus 1-10% (e.g., 1%, 2%, 3%, 4%, 5%, 6%, 7%, 8%, 9% or 10%) of the numerical value or end points of the numerical range. Thus, a value of 10 includes all numerical values from 9 to 11. All numerical ranges described herein include the endpoints of the range unless otherwise noted, and all numerical values in-between the end points, to the first significant digit.

As used herein, the term "policosanol" means one or more fatty alcohols. A policosanol can mean one fatty alcohol, such as vessel or solution containing 1-octacosanol. A policosanol can mean a mixture of two or more fatty alcohols, such as a vessel or solution containing 1-octacosanol, 1-triacontanol, and 1-dotriacontanol.

As used herein, the term "sterol" means one or more sterols. A sterol can mean one sterol, such as a vessel or solution containing ergosterol. A sterol can mean a mixture of two or more sterols, such as a vessel or solution containing a mixture of ergosterol, campesterol, stigmasterol, and β-sitosterol.

### Purification of Policosanol and Sterols from Lipid Compositions

In one aspect, the present disclosure provides a method of recovering a policosanol or a sterol from a composition, the method comprising: (a) dissolving the composition with a solvent, thereby forming a solution; (b) cooling the solution, thereby causing the policosanol or the sterol to precipitate from the solution; and (c) isolating the policosanol or the sterol; wherein the composition is a mixture comprising one or more fatty alcohols, aldehydes, fatty acids, and/or sterols.

In an embodiment, the solvent is ethyl acetate. In another embodiment, the ratio of the composition to the solvent is between 1:10 (w/v) to 1:30 (w/v). In a further embodiment, the ratio of the composition to the solvent is 1:20 (w/v).

In one embodiment, the solution is cooled to a temperature between 0 °C to -80 °C. In another embodiment, the solution is cooled to a temperature between -10 °C and -60 °C. In yet another embodiment, the solution is cooled to -30 °C.

In an embodiment, isolating the policosanol or the sterol occurs byway of a filtration step. In another embodiment, the filtration step comprises a vacuum. In a further embodiment, isolating the policosanol or the sterol occurs at a temperature of between -30-°C to 20 °C.

In one aspect, the present disclosure provides a composition obtained by the method of any one of the preceding paragraphs. In one embodiment, the composition contains a higher percentage of policosanol or sterol compared to a composition prior to undergoing the recovery methods described herein. In another embodiment, the composition contains 1 to 20 times more policosanol or sterol compared to a composition prior to undergoing the recovery methods described herein. In a further embodiment, the composition contains 2 to 15 times more policosanol or sterol compared to a composition prior to undergoing the recovery methods described herein. In yet another embodiment, the composition contains 3 to 11 times more policosanol or sterol compared to a composition prior to undergoing the recovery methods described herein.

In an embodiment, the policosanol comprises fatty alcohols. In an embodiment, the policosanol comprises 1-octacosanol, 1-triacontanol, and/or 1-dotriacontanol. In one embodiment, the sterol comprises ergosterol, campesterol, stigmasterol, and/or β-sitosterol.

In one embodiment, the policosanol has a melting point of between 25-30 °C. In another embodiment, the policosanol has a melting point of 28 °C. In one embodiment, the sterol has a melting point of between 140-150 °C. In another embodiment, the sterol has a melting point of 146 °C.

In an embodiment, the policosanol has a crystallization point of between 15-20 °C. In another embodiment, the policosanol has a crystallization point of between 17-18 °C. In one embodiment, the sterol has a crystallization point of between 140-150 °C. In another embodiment, the sterol has a crystallization point of 140 °C.

In one aspect, the present disclosure provides a method for treating or preventing a metabolic condition, the method comprising administering to a subject an effective amount of the composition of any one of the preceding paragraphs. In one embodiment, the metabolic condition is obesity, dyslipidemia, diabetes, or a cardiovascular condition.

In one aspect, the present disclosure provides the composition for use in the treatment of a metabolic condition. In one embodiment, the metabolic condition is obesity, dyslipidemia, diabetes, or a cardiovascular condition.

### EXAMPLES

### Example 1. Winterization of Lipid Extracts

Sugarcane is used as a feedstock for industrial fermentation applications. Purified filter cake (PFC) from processed sugarcane was characterized and found to contain 1-octacosanol as the major fatty alcohol in a mixture that included other fatty alcohols, aldehydes, and fatty acids. In addition, distillate residue from a farnesene fermentation process (PFDR) was characterized and found to contain different sterols, with ergosterol being the most abundant, in a mixture constituted mostly of terpenes.

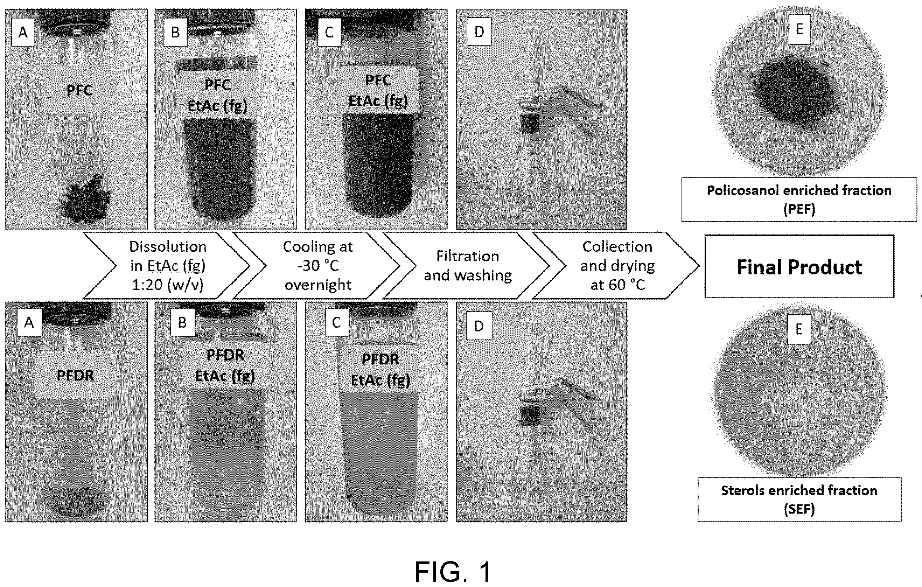

In a glass container with a lid, the PFC and PFDR lipid extracts (FIG. 1A) were each mixed with ethyl acetate (EtAc) food grade at a 1:20 (w/v) proportion and stirred (manual, magnetic, or mechanic stirring) until complete dissolution (FIG. 1B). Then, the glass containers were left at room temperature for 2h. In a second glass container, an equal volume of EtAc food grade was added, and reserved for future washing of the isolate. Both containers were placed at -30 °C and kept overnight (FIG. 1C). Then, the first container was passed through filter paper, under vacuum and in cold conditions (while the sample was still cold) (FIG. 1D). When filtration was complete, the cold solvent reserved in the second container was used to wash the solid trapped in the filter paper, by pouring it over the solid and mixing it with a glass rod, maintaining the filtration conditions. The solid fraction (product) was collected and dried at 60°C until constant weight. The solid fraction obtained from PFC was denominated as policosanol enriched fraction (PEF) and the solid fraction obtained from PFDR as sterols enriched fraction (SEF) (FIG. 1E). The resulting filtrate was evaporated in a rotatory evaporator to recover the solvent (90 - 95%) for eventual reuse.

### Example 2. Isolate Yield and Purity

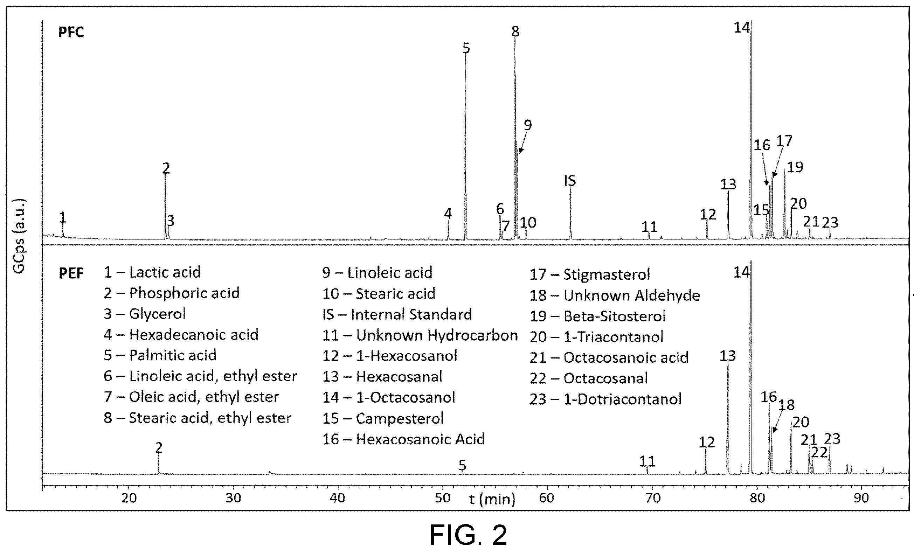

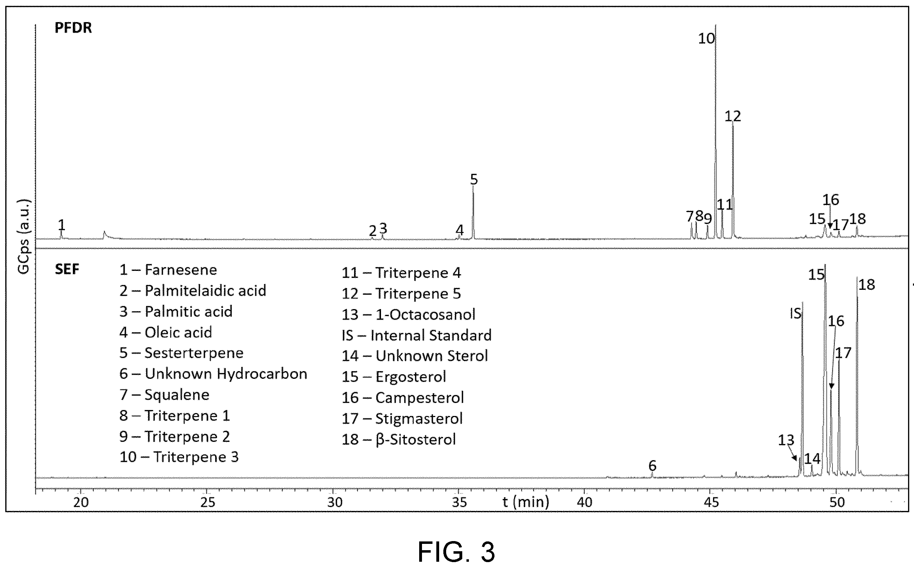

The isolation yields were of 40.93 ± 0.25% for PEF and of 3.03 ± 0.69% for SEF. The composition of these enriched fractions was confirmed by GC-MS analysis. The composition of both PFC and PEF lipid extracts and PEF and SEF enriched fractions are described in FIG. 2 and FIG. 3.

The overlay of the GC-MS chromatograms in FIG. 2 clearly shows a concentration of fatty alcohols (peaks 12. 1-Hexacosanol, 14. 1-Octacosanol, 20. 1-Triacontanol and 23. 1-Dotriacontanol) and some aldehydes in the PEF isolate, without the fatty acids and fatty esters found in the PFC lipid extract. Similarly, in FIG. 3 it is also evident that the sterols (peaks 15. Ergosterol, 16. Campesterol, 17. Stigmasterol and 18. β-Sitosterol) that were present in the PFDR lipid extract were completely isolated in SEF, while not being possible to detect any other component of the PFDR lipid extract in the SEF isolate.

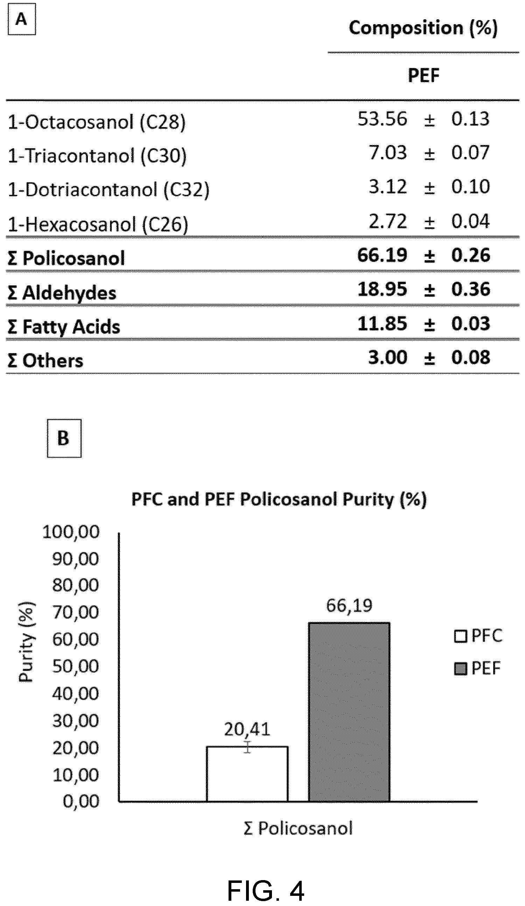

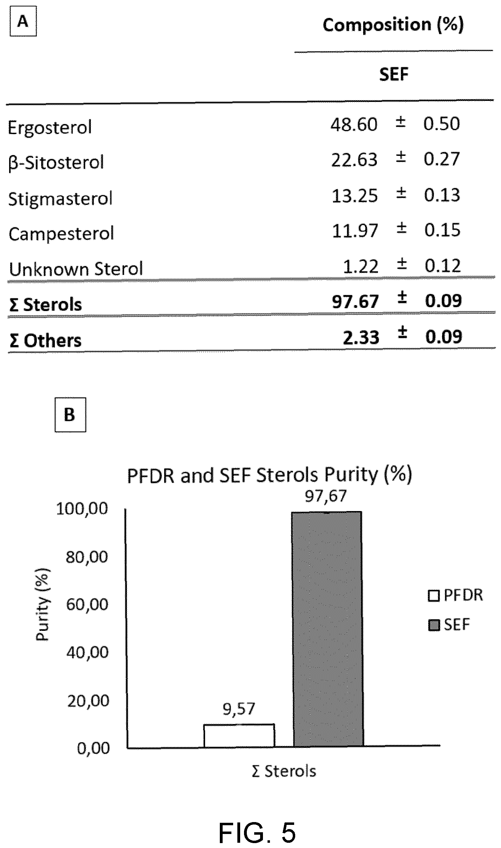

Furthermore, GC-MS analysis also allowed a quantitative characterization of the isolates (by area percentage) and to access the purity degree of policosanol in PEF and sterols in SEF. Moreover, using the same quantification method for the PFC and PFDR, the purity degree of policosanol and sterols, respectively, are also showed. The results are presented in FIG. 4 for PFC/PEF and in FIG. 5 for PFDR/SEF.

Concerning the PEF composition (FIG. 4A), it was mainly formed of policosanol (66.19 ± 0.26 %), with lower amounts of aldehydes (18.95 ± 0.36 %), fatty acids (11.85 ± 0.03 %) and 3.00 ± 0.08 % of other compounds. Within policosanol, the major fatty alcohol was 1-octacosanol at 53.56 ± 0.13 %. Comparing the policosanol purity degree between the lipid extract PFC (20.41 ± 2.12 %) and the respective isolate PEF (66.19 ± 0.26 %) (FIG. 4B), an approximate 3-fold increase was noted, confirming the success of the isolation.

Regarding the composition of SEF (FIG. 5A), it was a mixture of sterols (97.67 ± 0.09 %) where ergosterol was the major compound (48.60 ± 0.50 %), with a very low amount of other components (2.33 ± 0.09 %). Comparing the sterols purity between PFDR (9.57 ± 0.23 %) and SEF (97.67 ± 0.09 %) (FIG. 5B), an approximate 10-fold increase was noted, once again confirming the suitability of the method for the isolation of sterols.

### Example 3. Thermal Properties

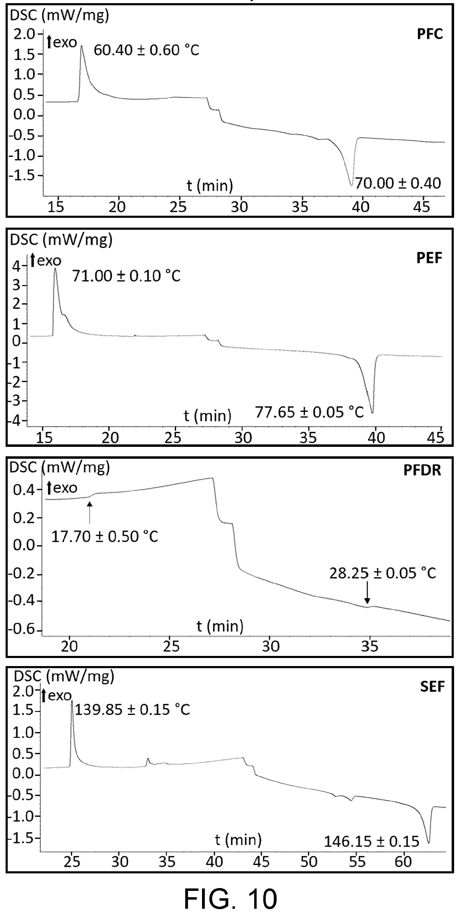

Thermal properties of the lipid extracts (PFC and PFDR) and the isolates (PEF and SEF) were characterized by Differential Scanning Calorimetry (DSC) (FIG. 10). The results are shown below in Table 1.

**Table 1. DSC analysis results (melting and crystallization points).**

| | **Melting (°C)** | **Cristalization (°C)** |
|---|---|---|
| | **Mean** ± **SD** | **Mean** ± **SD** |
| **PFC** | 70.00 ± 0.40 | 60.40 ± 0.60 |
| **PEF** | 77.65 ± 0.05 | 71.00 ± 0.10 |
| **PFDR** | 28.25 ± 0.05 | 17.70 ± 0.50 |
| **SEF** | 146.15 ± 0.15 | 139.85 ± 0.15 |

The PFC lipid extract is a wax with a multivariate composition [free fatty acids (FFA), policosanol, fatty esters, and sterols]. Despite this, DSC analysis revealed a single melting point (70.00 ± 0.40 °C) and a single crystallization point (60.40 ± 0.60 °C), showing wide and intense transition peaks, that probably include all the transitions of each compound. Similarly, the corresponding PEF isolate showed a melting point of 77.65 ± 0.05 °C and a crystallization point of 71.00 ± 0.10 °C, revealing less wide transition peaks since this sample was an extract from PFC and therefore had a less diverse composition. Regarding its main compound, 1-octacosanol (melting point of 83.2 - 83.4 °C), and comparing the melting points of 1-octacosanol and PEF, it can be said that they are close but that the remaining compounds present in PEF affect (*i.e.,* decrease) its melting point.

PFDR is mainly composed of terpenes that act as a solvent/dispersant towards the sterols and FFA that compose this sample, giving PFDR an appearance of a slightly viscous liquid with some solids dispersed in it. DSC analysis revealed that in this mixture, the dispersed solid compounds were able to melt when heated to 28.25 ± 0.05 °C; moreover, along the cooling step of DSC analysis, it was possible to identify the crystallization temperature of the solids as being 17.70 ± 0.50 °C, both detected as discrete transitions.

Regarding the DSC analysis of SEF, a white solid composed of sterols (-97.7%), two intense and wide peaks were detected, one of them endothermic, associated with the sterols melting and the other, exothermic, related to their crystallization. The shape of the peaks suggests that it is a result of the accumulation of various transitions, *i.e.,* the melting or crystallization of the various sterols that compose SEF; then the transition temperature measured was also probably a result of a balanced combination of the transition temperatures of each sterol. The melting temperature of SEF was 146.15 ± 0.15 °C and the respective crystallization temperature was 139.85 ± 0.15 °C. As described above, the main sterols present in this sample are ergosterol (-48.6%) and β-sitosterol (-22.6%); these compounds have melting points of 168 °C and 140 °C, respectively. Comparing the melting points of SEF, ergosterol and β-sitosterol, it was noted that the melting temperature of SEF was found between the melting points of its main sterols.

### Example 4. Structural and Functional Properties

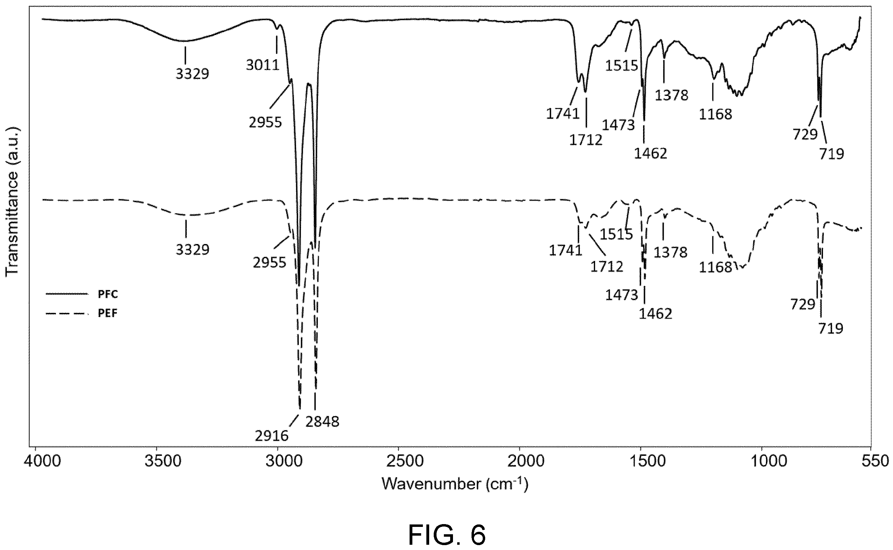

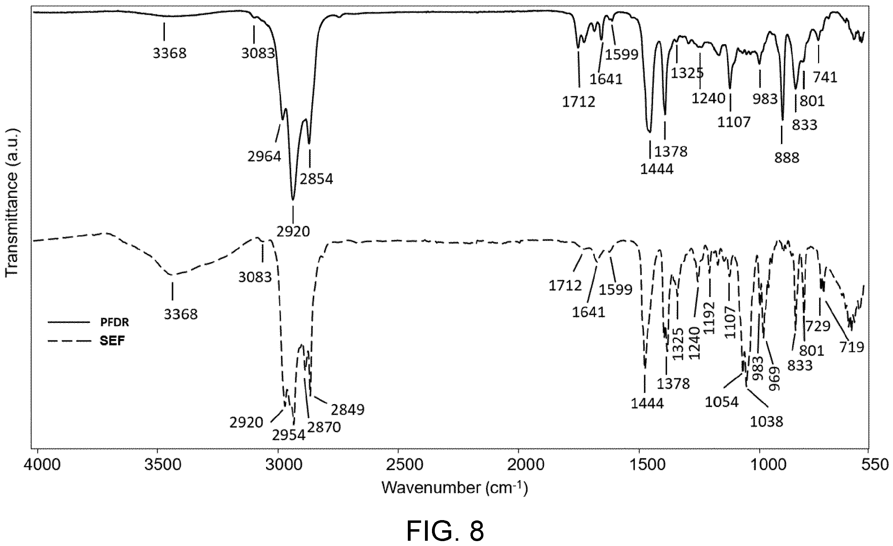

The winterization process described herein allows the isolation of compounds or a family of compounds of interest from lipid extracts with a complex composition. Fourier transform infrared spectroscopy (FTIR) is a sensitive and precise analytical technique that quickly provides an understanding of the main functional groups present in a sample. The lipid extracts (PFC and PFDR) as well as the respective isolates (PEF and SEF) were analyzed by FTIR-ATR (FTIR with an attenuated total reflectance accessory). The FTIR-ATR spectra of each extract and isolate were overlapped for comparison of their functional properties and better understanding of the success of the isolation. The overlapping of the FTIR-ATR spectra is represented in FIG. 6 (PFC vs. PEF) and in FIG. 8 (PFDR vs SEF), and the identification of the FTIR vibrational bands was based on literature (Socrates, 2001, doi: 10.1002/irs.1238).

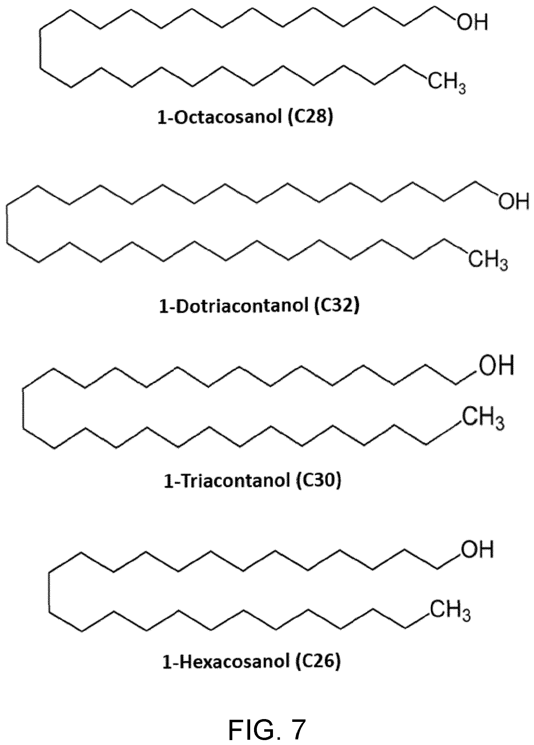

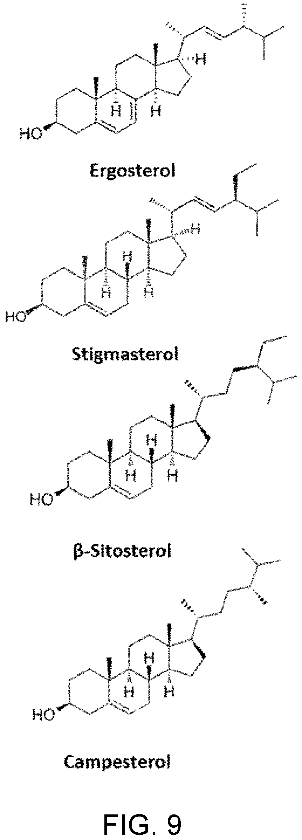

To better understand the structural differences described below, FIG. 7 and FIG. 9 represent the structures of the main constituents of PEF and SEF, respectively.

The overlay of the FTIR-ATR spectra of PFC vs PEF revealed a similar profile with particular differences. In common, both samples presented alcohols (vibrational bands at 3329 cm⁻¹ and 1515 cm⁻¹ related to the -OH and C-O stretching in alcohols), aldehydes, and hydrocarbons (vibrational bands between 2955- 2848 cm⁻¹ associated with C-H stretching in alkanes, alkenes and aldehydes, the bands at 1473, 1462 and 1378 cm⁻¹ respectively corresponding to the C-C stretching, -C-H deformation and CH₃ deformation in aliphatic chains and the vibrational bands at 729 and 719 cm⁻¹, typical of -C-H bending vibration in alkanes). The major differences were related to the almost total elimination of FFAs and esters during the PEF isolation process. Vibrational bands at 1741 and 1712 cm⁻¹ that correspond to C=O stretching vibration of the carbonyl group in fatty esters and fatty acids, respectively, were very well defined in PFC but not in PEF. Similarly, the vibrational bands at 3011 and 1168 cm⁻¹, associated with the =C-H stretching and bending in alkenes, were almost untraceable in PEF FTIR spectrum, probably due to the elimination of unsaturated FFAs and esters.

As discussed previously in Example 2, the lipid extract PFDR was composed of terpenes, free fatty acids, fatty alcohols, and sterols. After the winterization procedure, the isolate SEF was mainly composed of sterols. The overlay of the FTIR-ATR spectra of PFDR vs SEF (FIG. 8) visualizes the differences in the composition of the samples, reinforcing the success of the isolation method. In common, the FTIR-ATR spectra presented some vibrational bands that are transverse to organic molecules such as the band at 3083 cm⁻¹ related to the =C-H stretching vibration; the bands between 2964-2849 cm⁻¹ associated with the C-H stretching; and the bands at 1444 cm⁻¹ and 1378 cm⁻¹ corresponding to the C-H and the CH₃ deformation; all of them in aliphatic chains of alkanes and alkenes. The main differences relate to the drastic decrease in intensity of some vibrational bands, for instance, at 1641 cm⁻¹ and 1599 cm⁻¹ associated with C=C stretching vibration, at 888 cm⁻¹ associated with the C=C bending, and at 741 cm⁻¹ related to the C-H bending vibrations in alkenes. This is probably due to the elimination of terpenes (farnesene, squalene, sesterterpene, and triterpenes, where the later belong to the squalene family), that in this case were linear polyunsaturated alkenes, during SEF isolation. Similarly, the FTIR vibrational bands at 1712 and 1107 cm⁻¹ respectively corresponding to the C=O stretching in carboxylic acids and the C-C stretching in alkanes aliphatic chains, also decreased in intensity from PFDR to SEF FTIR-ATR spectra. In this case, it was in accordance with the elimination of FFAs (saturated and monounsaturated) during the isolation process.

On the other hand, the increase in intensity and resolution of some FTIR vibrational bands from PFDR to SEF spectra was also detected, namely at 3368 cm⁻¹ associated with the -OH stretching vibration and at 1325, 1240 and 1192 cm⁻¹ associated with the C-O stretching vibration in the alcohol functional group. This was a consequence of the isolation of sterols in SEF. Furthermore, the increased intensity in bands at 1054 and 1038 cm⁻¹ related to =C-H bending and at 983 and 969 cm⁻¹ associated with the C=C bending in alkenes were also probably related to the isolation of sterols in SEF, since the basic structure of this family of molecules is an unsaturated polycyclic structure (FIG. 9). Moreover, other vibrational bands that increased in intensity in the SEF FTIR-ATR spectrum were the bands at 833 and 801 cm⁻¹ correlating to the C-H bending vibrations in alkenes and the bands at 729 and 719 cm⁻¹ associated with the C-H bending vibrations in alkanes. These results follow the latter, once these vibrations can be associated with the different substituent groups that characterize each of the sterols, being that the ergosterol and the stigmasterol substituents are monounsaturated aliphatic chains (alkenes) and that the β-sitosterol and campesterol substituents are saturated aliphatic chains (alkanes).

### Example 5. Toxicity of the Enriched Fractions in Hepatocytes

The cytotoxicity of the isolates (PEF and SEF) was assessed in hepatocytes, HepG2 (HB-8065^{™}) cells, to evaluate their biocompatibility. As shown in FIG. 10, both fractions, PEF and SEF, are biocompatible with hepatocytes at 0.05 mg/mL (*i.e.,* below 30% of metabolic inhibition), which corresponds to its solubility limit in aqueous medium.

### Other Embodiments

All publications, patents, and patent applications mentioned in this specification are incorporated herein by reference to the same extent as if each independent publication or patent application was specifically and individually indicated to be incorporated by reference.

While the invention has been described in connection with specific embodiments thereof, it will be understood that it is capable of further modifications and this application is intended to cover any variations, uses, or adaptations of the invention following, in general, the principles of the invention and including such departures from the invention that come within known or customary practice within the art to which the invention pertains and may be applied to the essential features hereinbefore set forth, and follows in the scope of the claims.

Other embodiments are within the claims.

## Claims

1. A method of recovering a policosanol or a sterol from a composition, the method comprising:
(a) dissolving the composition with a solvent, thereby forming a solution;
(b) cooling the solution, thereby causing the policosanol or the sterol to precipitate from the solution; and
(c) isolating the policosanol or the sterol;
wherein the composition is a mixture comprising one or more fatty alcohols, aldehydes, fatty acids, and/or sterols.

2. The method of claim 1, wherein the solvent is ethyl acetate.

3. The method of claim 1 or 2, wherein the ratio of the composition to the solvent is between 1:10 (w/v) to 1:30 (w/v), preferably wherein the ratio of the composition to the solvent is 1:20 (w/v).

4. The method of any one of claims 1 to 3, wherein the solution is cooled to a temperature between 0 °C to -80 °C, preferably wherein the solution is cooled to a temperature between -10 °C and -60 °C, more preferably wherein the solution is cooled to -30 °C.

5. The method of any one of claims 1 to 4, wherein isolating the policosanol or the sterol occurs by way of a filtration step.

6. The method of claim 5, wherein the filtration step comprises a vacuum.

7. The method of any one of claims 1 to 6, wherein isolating the policosanol or the sterol occurs at a temperature of between -30-°C to 20 °C.

8. A composition obtained by the method of any one of claims 1 to 7.

9. The composition of claim 8, wherein the composition contains a higher percentage of policosanol or sterol compared to the composition of claim 1, preferably 1 to 20 times more, more preferably 2 to 15 times more, most preferably 3 to 11 times more.

10. The composition of any one of claims 8 to 9, wherein the policosanol comprises fatty alcohols.

11. The composition of claim 10, wherein the policosanol comprises 1-octacosanol, 1-triacontanol, and/or 1-dotriacontanol.

12. The composition of any one of claims 8 to 11, wherein the sterol comprises ergosterol, campesterol, stigmasterol, and/or β-sitosterol.

13. The composition of any one of claims 8 to 12, wherein the policosanol has a melting point of between 25-30 °C, preferably of 28 °C.

14. The composition of any one of claims 8 to 13, wherein the sterol has a melting point of between 140-150 °C, preferably of 146 °C.

15. The composition of any one of claims 8 to 14, wherein the policosanol has a crystallization point of between 15-20 °C, preferably between 17-18 °C.

16. The composition of any one of claims 8 to 15, wherein the sterol has a crystallization point of between 140-150 °C, preferably of 140 °C.

17. A composition of any one of claim 8 to 16 for use in the treatment of a metabolic condition.

18. The composition for use according to claim 17, wherein the metabolic condition is obesity, dyslipidemia, diabetes, or a cardiovascular condition.
